# EUROPEAN PATENT APPLICATION

(11) **EP 4 786 587 A1**
(43) Date of publication of application: **05.08.2026**
(21) Application number: 24870937.0
(22) Date of filing: 27.09.2024
(51) Int. Cl.: C12N 15/11, C12N 15/10, B01J 13/02, C12P 21/00, C12N 15/67

(54) **DNA MICROSPHERE, PREPARATION METHOD THEREFOR, AND USE THEREOF**

(30) Priority: 28.09.2023 CN 202311285847
(71) Applicant: Kangma-Healthcode (Shanghai) Biotech Co., Ltd, Pudong New Area Shanghai 201321 (CN)
(72) Inventor: GUO, Min, Shanghai 201321 (CN); ZHOU, Zijian, Shanghai 201321 (CN); YU, Quanhao, Shanghai 201321 (CN); WANG, Yanmin, Shanghai 201321 (CN); LI, Ning, Shanghai 201321 (CN); YU, Xue, Shanghai 201321 (CN)
(74) Representative: Dai, Simin
(86) International application number: PCT/CN2024/121645
(87) International publication number: WO 2025/067399

(57) **Abstract**

Provided is a DNA microsphere, comprising: a microsphere body, a polymer connected to the surface of the microsphere body, and DNA combined with the polymer. The provided DNA microspheres can be stably used in an in-vitro protein synthesis reaction, significantly reducing the protein preparation cost and providing a potential method for large-scale industrial protein production, facilitating the research, development and production of protein drugs.

## Description

### TECHNICAL FIELD

The present disclosure belongs to the field of biochemical technology, and specifically relates to a biological microsphere, and a preparation method therefor and the use thereof.

### BACKGROUND

DNA template plays a crucial role in in vitrogene expression systems, but its high cost and low utilization rate have consistently constrained the industrialization process of related technologies. The synthesis of DNA template primarily relies on chemical synthesis or bacterial amplification, which is costly and difficult to achieve large-scale production, failing to meet industrialization requirements. In addition, DNA templates are difficult to reuse in in *vitro* transcription-translation reactions, resulting in resource waste.

Reusable DNA templates can maximize the role of templates and achieve resource recycling. Therefore, it is particularly necessary to develop low-cost and reusable DNA templates. The development of artificial synthetic biology has provided new insights for the construction of this novel template. Compared to DNA recycling technology, linear template technology and other methods that connect DNA templates to microspheres or other solid supports enable template separation, elution, and reuse, offering advantages such as ease of operation, low cost, and strong functional extensibility.

An in vitrobiosynthesis system refers to the in vitro efficient synthesis of specific chemical molecules or biological macromolecules (DNA, RNA, or proteins) within a lysate system derived from bacterial, fungal, plant cells, or animal cells by adding exogenously encoded nucleic acids (DNA or, RNA), substrates, and energy sources.

A common example of an in vitrobiosynthesis system is the in vitroprotein synthesis system, also known as the cell-free protein synthesis system, which employs exogenous mRNA or DNA templates and cell lysates to accomplish rapid and efficient translation of exogenous recombinant proteins; for another example, the in vitroRNA synthesis system (IVT).

Some in vitroprotein synthesis systems are in vitrotranscription-translation coupled systems (in vitrotranscription-translation system, abbreviated as IVTT), which utilize a DNA template to transcribe mRNA intermediates via RNA polymerase, and then employ amino acids, ATP, and other components to achieve efficient one-step translation of exogenous proteins. Currently, common commercial in vitroprotein expression systems include the Escherichia coli system (Escherichia coli extract, ECE), the Rabbit Reticulocyte Lysate (RRL) system, the Wheat Germ Extract (WGE) system, the Insect Cell Extract (ICE) system, and the human-derived system.

Compared with conventional in vivo recombinant expression systems, the cell-free protein synthesis system offers various advantages, such as the ability to express special proteins that are toxic to cells or contain unnatural amino acids (e.g., D-amino acids), the ability to directly use PCR products as templates for parallel synthesis of multiple proteins, and the facilitation of high-throughput drug screening and proteomics research.

Current in vitro synthesis systems have the following limitations:
1. All existing in vitrosynthesis systems rely on mRNA or DNA templates, and the template amount is typically far greater than the mass of the final synthesized protein, which limits the industrial application of in vitroprotein synthesis technology and its potential for low cost. Currently, the cost of in *vitro* synthesized proteins remains high, making it difficult to meet the demands of large-scale industrial production.
2. Currently commercialized in vitro protein synthesis systems mainly include prokaryotic systems (such as Escherichia coli) and eukaryotic systems (such as insect cells, mammalian cells, yeast, etc.). In contrast, prokaryotic systems such as Escherichia coli are easier to culture and manipulate, offering high yields, but the synthesized proteins exhibit lower activity and correctness compared to eukaryotic systems. Proteins synthesized by eukaryotic cell systems possess higher activity and post-translational modification, but they come with high costs and difficult to produce on a large-scale production basis.
3. The application scope of in vitroprotein synthesis technology is relatively limited, mainly concentrated in the field of drug development, for producing recombinant proteins for in vivo studies or therapeutic protein drugs. The application potential in industrial production and other fields remains to be explored.
4. Currently, the mainstream technology for global industrial protein production remains the cell fermentation-based method. Compared with cell fermentation, in vitro protein synthesis technology offers advantages such as simple operation, no risk of viral contamination, and high yield, but it has higher costs and a lower degree of industrialization, making it difficult to replace the dominant position of cell fermentation in industrial protein production.

In summary, although in vitroprotein synthesis technology has high potential, its current level of industrialization and large-scale application remains relatively low, with the main obstacles being high costs and strong template dependence. Breakthroughs in related technologies will facilitate broader application of in vitro protein synthesis technology in protein drug development and industrial production.

Current status of existing DNA template technologies:
1. The DNA template is primarily used to guide in vitrotranscription and translation reactions for synthesizing RNA and proteins. Commonly used DNA templates include plasmid DNA, linear DNA, and circular DNA. These DNA templates need to be obtained through bacterial transformation, PCR amplification, or synthesis.
2. Currently, the acquisition of DNA templates primarily relies on chemical synthesis or bacterial transformation amplification, which is costly and operationally cumbersome, making it difficult to achieve industrialization and large-scale application. Next-generation gene synthesis technologies, such as gene synthesis chip technology, have reduced costs. However, for the amounts of DNA templates required for industrial production, the cost remains relatively high.
3. In in vitrotranscription and translation systems, in vitroreactions typically require large amounts of DNA template (exceeding the mass of the final product), which limits the application of related technologies on an industrial scale. The method for reusing or continuously utilizing DNA templates can reduce costs and achieve industrial production.
4. In recent years, some novel methods have achieved the reuse or continuous utilization of DNA templates, such as DNA recycling technology, linear expression template technology, isothermal amplification, etc. The emergence of these new methods has provided new insights for the large-scale utilization of DNA templates in industrial applications. However, the degree of industrialization of related technologies remains relatively low, and the cost advantage needs to be further improved.

Advantages and disadvantages of other DNA template reuse or continuous utilization technologies:
1. Advantages of DNA recycling technology: DNA template can be continuously reused, reducing the required template amount; this technology is simple in operation, and easy to implement.

Disadvantages: The design of DNA cycling strategies is relatively complex, as different templates exhibit varying cycling functions and stability; DNA cycling amplification is prone to introducing errors, requiring highly sensitive detection methods.

2. Advantages of linear expression template technology: Linear DNA can be easily obtained through chemical synthesis at a relatively low cost; this technology exhibits certain template stability and can be utilized continuously.

Disadvantages: Linear DNA exhibits lower stability in in vitrotranscription-translation reactions and has reduced utilization efficiency compared to DNA cycling; special treatment is required to prevent linear DNA from degradation in the reaction.

3. Advantages of isothermal amplification: The operation for reaction is simple, utilizing PCR to automatically perform DNA amplification at a constant temperature with high efficiency.

Disadvantages: This technology requires specialized primers and DNA polymerase, resulting in higher costs; the amplification of DNA sequences requires the design and introduction of circularization sequences or restriction sites, which is relatively cumbersome; the yield of amplified DNA is difficult to precisely control and exhibits certain variations.

4. Advantages of CRISPR-Cas9 gene editing technology: The DNA template can be directionally modified to achieve reuse; the operation is relatively simple, and based on the principle of enzymatic cleavage of DNA-RNA complexes.

Disadvantages: this technology requires gRNA design and synthesis, resulting in higher costs; it is difficult to completely avoid generating a certain proportion of non-specific editing products.

### CONTENT OF THE PRESENT INVENTION

In view of the fact that existing in vitroprotein synthesis systems are difficult to meet the needs of daily biological research and industrialization, the present disclosure provides a reusable DNA template that can be stably used in an in-vitro protein synthesis reaction, significantly reducing the protein preparation cost and providing a potential method for large-scale industrial protein production, facilitating the research, development and production of protein drugs.

To this end, the present application has discovered through research that by linking the DNA template to microspheres, a novel DNA template microsphere can be prepared, and this DNA microsphere can serve as a reusable DNA template. This solution can not only reduce DNA template costs to achieve industrial production, but also opens up new research directions (such as directed cell evolution and functional regulation) in the field of artificial synthetic biology, bringing new opportunities for the widespread application of DNA template technology (including gene therapy and synthetic biology research).

Specifically, we selected biocompatible microspheres as the solid support to link linear template DNA via chemical bond, and optimized reaction conditions to achieve high linking efficiency and template stability. By verifying technical specifications such as its stability in reactions, number of uses, and efficiency for reuse, it was demonstrated that this novel DNA microsphere has significant application potential in industrial production and reducing gene expression costs.

A first aspect of the present disclosure provides a DNA microsphere, comprising: a microsphere body, a polymer connected to the surface of the microsphere body, and DNA bound to the polymer.

Further preferably, the polymer binds to DNA via a functional group.

Further preferably, the functional group is selected from a carboxyl group, a hydroxyl group, an amino group, a sulfhydryl group, a salt form of a carboxyl group, a salt form of an amino group, an ester group, or a combination of the foregoing functional groups.

Further preferably, the DNA is modified by the functional group.

Further preferably, the DNA is obtained by amplification of the DNA primer sequence.

Further preferably, the length of the DNA primer sequence is 6-30 bp; preferably 9-21 bp.

Further preferably, the DNA primer sequence is bound to the polymer via the functional group prior to the amplification.

Further preferably, the DNA primer sequence is bound to the polymer via the functional group for the amplification and then connected to the surface of the microsphere body to obtain the DNA microsphere.

Further preferably, the polymer has a branched chain.

Further preferably, the polymer binds to DNA through the branched chain.

Further preferably, the polymer is directly connected to the outer surface of the microsphere body or indirectly connected to the outer surface of the microsphere body via a linking group.

Further preferably, one end of the linking group is connected to the microsphere, and the other end is linked to the polymer by binding to an active group (such as an amino group, a carboxyl group, etc.) at the terminus of the polymer.

Further preferably, the outer surface of the microsphere body has at least one linear polymer with branched chains, wherein one end of the linear polymer with branched chains is connected to the outer surface of the microsphere body, and the remaining part is free from the outer surface of the microsphere body; the branched chain of the linear polymer is bound to DNA.

Further preferably, the linear polymer is directly immobilized on the outer surface of the microsphere body or indirectly immobilized on the outer surface of the microsphere body via a linking group.

Further preferably, the linking group is selected from one or a combination of substituted or unsubstituted siloxy group, amide group, ester group, carbonyl group, alkenyl group, alkynyl group, alkylene group, ether group, and thioether group.

Further preferably, the surface of the microsphere body contains silicon or silicon oxide.

Further preferably, the material of the microsphere body is selected from a glass material, a ceramic material, or a magnetic material.

Further preferably, the microsphere body is a magnetic microsphere.

Further preferably, the microsphere is selected from a glass material, a ceramic material, or a magnetic material.

Further preferably, the chemical composition of the magnetic material includes any one of or any combination of iron compound, iron alloy, zinc oxides, manganese oxides, gadolinium oxides, cobalt compound, nickel compound, nickel alloy, manganese oxides, manganese alloy, zinc oxides, gadolinium oxides, and chromium oxides.

Further preferably, the chemical composition of the magnetic material includes any one of or any combination of iron oxides, zinc oxides, manganese oxides, gadolinium oxides, cobalt oxides, nickel oxides, manganese oxides, zinc oxides, gadolinium oxides, and chromium oxides.

Further preferably, the chemical component of the magnetic material includes any one of or any combination of iron oxide, iron, cobalt, Co²⁺, iron nitride, Mn₃O₄, GdO, nickel, and Ni²⁺; wherein, the iron oxides are preferably Fe₃O₄, γ-Fe₂O₃, or a combination thereof.

Further preferably, the chemical component of the magnetic material includes any one of or any combination of Fe₃O₄, γ-Fe₂O₃, iron nitride, Mn₃O₄, AlNi(Co), FeCr(Co), FeCrMo, FeAlC, AlNi(Co), FeCrCo, ReCo, ReFe, PtCo, MnAlC, CuNiFe, AlMnAg, MnBi, FeNi(Mo), FeSi, FeAl, FeNi(Mo), FeSiAl, BaO·6Fe₂O₃, SrO·6Fe₂O₃, PbO·6Fe₂O₃, and GdO.

Further preferably, the microsphere is selected from a glass microsphere, a ceramic microsphere or a glass-ceramic microsphere.

Further preferably, the microsphere may be hollow.

Further preferably, the size of the microsphere body is selected from any one of the following particle size scales or a range between any two particle size scales: 0.1 µm, 0.15 µm, 0.2 µm, 0.25 µm, 0.3 µm, 0.35 µm, 0.4 µm, 0.45 µm, 0.5 µm, 0.55 µm, 0.6 µm, 0.65 µm, 0.7 µm, 0.75 µm, 0.8 µm, 0.85 µm, 0.9 µm, 0.95 µm, 1 µm, 1.5 µm, 2 µm, 2.5 µm, 3 µm, 3.5 µm, 4 µm, 4.5 µm, 5 µm, 5.5 µm, 6 µm, 6.5 µm, 7 µm, 7.5 µm, 8 µm, 8.5 µm, 9 µm, 9.5 µm, 10 µm, 15 µm, 20 µm, 25 µm, 30 µm, 35 µm, 40 µm, 45 µm, 50 µm, 55 µm, 60 µm, 65 µm, 70 µm, 75 µm, 80 µm, 85 µm, 90 µm, 95 µm, 100 µm, 150 µm, 200 µm, 250 µm, 300 µm, 350 µm, 400 µm, 450 µm, 500 µm, 550 µm, 600 µm, 650 µm, 700 µm, 750 µm, 800 µm, 850 µm, 900 µm, 950 µm, and 1000 µm; the diameter dimensions are average values.

Further preferably, the diameter of the microsphere body is selected from 0.1-10 µm.

Further preferably, the diameter of the microsphere body is selected from 0.2-6 µm.

Further preferably, the diameter of the microsphere body is selected from 0.4-5 µm.

Further preferably, the diameter of the microsphere body is selected from 0.5-3 µm.

Further preferably, the diameter of the microsphere body is selected from 0.2-1 µm.

Further preferably, the diameter of the microsphere body is selected from 0.5-1 µm.

Further preferably, the average diameter of the microsphere body is 200 nm, 250 nm, 300 nm, 350 nm, 400 nm, 450 nm, 500 nm, 550 nm, 600 nm, 650 nm, 700 nm, 750 nm, 800 nm, 850 nm, 900 nm, 950 nm, or 1000 nm, with a deviation of ±20%, more preferably ±10%.

Further preferably, the backbone of the linear polymer is a polyolefin backbone or an acrylic polymer backbone.

Further preferably, the linear backbone of the polymer is a polyolefin backbone and is provided by the backbone of an acrylic polymer.

Preferably, the average molecular weight (MW) of the polymer is 1000-5000000; more preferably, the MW is 1000-100000; further preferably, the MW is 2000-50000; still further preferably, the MW is 3000-10000.

Further preferably, the monomer unit of the acrylic polymer is one of or a combination of acrylic acid, acrylate salt, acrylate ester, methacrylic acid, methacrylate salt, and methacrylate ester.

A second aspect of the present disclosure provides a preparation method for the DNA microsphere according to the first aspect of the present disclosure.

Further preferably, the preparation method comprises the following steps: (1) chemically modifying the microsphere body with an aminated silane coupling agent to introduce an amino group onto the outer surface of the microsphere body, thereby forming an amino-modified microsphere A; (2) modifying a DNA primer sequence to obtain a modified DNA primer sequence; (3) binding the modified DNA primer sequence to a polymer, followed by amplification to obtain a polymer-modified DNA; (4) reacting the polymer-modified DNA with the amino-modified microsphere A to obtain the DNA microsphere.

Further preferably, the aminated silane coupling agent is selected from 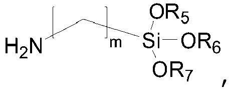 wherein R₅ to R₇ are each independently selected from C₁-C₁₀ alkyl, and m is 1-10.

Further preferably, m is preferably 1, 2, 3, 4, 5, 6, 7, 8, 9, or 10.

Further preferably, R₅ to R₇ are each independently selected from alkyl having a number of carbon atoms of 1, 2, 3, 4, 5, 6, 7, 8, 9, or 10.

Further preferably, the aminated silane coupling agent is selected from 3-aminopropyltriethoxysilane or 3-aminopropyltrimethoxysilane.

Further preferably, in step (2), the DNA primer sequence is modified with the amino group at the 5' end; more preferably, the DNA primer sequence is modified with the amino group at C2-C20 position of the 5' end; still more preferably, the DNA primer sequence is modified with the amino group at the C5-C10 position of the 5' end; most preferably, the DNA primer sequence is modified with the amino group at the C6 position of the 5' end.

Further preferably, the length of the DNA primer sequence is 6-30 bp; preferably 9-21 bp.

Further preferably, the DNA primer sequence is GGCGTAGAGGAT.

Further preferably, the DNA primer sequence is bound to the polymer via the functional group prior to the amplification.

Further preferably, the DNA primer sequence is bound to the polymer via the functional group for the amplification and then connected to the surface of the microsphere body to obtain the DNA microsphere.

Further preferably, the modification in step (2) is the reaction of the DNA sequence with the polyolefin polymer.

Further preferably, the polyolefin polymer is selected from one or more of substituted or unsubstituted polypropylene, polyacrylic acid, polyacrylate salt, polyacrylate ester, or polyacrylamide.

Further preferably, the substituent is selected from C1-C20 alkyl.

Further preferably, the polyolefin polymer is selected from polyacrylic acid, polyacrylate salt, polymethacrylic acid or polymethacrylate salt.

Further preferably, in step (4), before reacting the polymer-modified DNA with the microsphere A, the system containing the microsphere A is subjected to ultrasonic concussion.

Further preferably, the ultrasonic concussion is performed for 1-30 min, more preferably 5-20 min; particularly preferably 5-10 min.

A third aspect of the present disclosure provides a use of the DNA microsphere according to the first aspect of the present disclosure.

Further preferably, the DNA microsphere can be used as a DNA template.

Further preferably, the DNA template is a DNA template for the protein of interest (or exogenous protein).

Further preferably, the protein of interest is selected from the following group: fluorescent protein, or luciferase (such as firefly luciferase), green fluorescent protein, yellow fluorescent protein, aminoacyl-tRNA synthetase, glyceraldehyde-3-phosphate dehydrogenase, catalase, actin, variable region of antibody, luciferase mutant, α-amylase, enterocin A, hepatitis C virus E2 glycoprotein, proinsulin, interferon αA, cytokine, interferon α2b, interleukin-1β, lysozyme, serum albumin, single-chain antibody fragment (scFV), transthyretin, tyrosinase, xylanase, or a combination thereof.

A fourth aspect of the present disclosure provides a use of the DNA microsphere according to the first aspect of the present disclosure as a DNA template in RNA transcription or protein synthesis.

Further preferably, the RNA transcription or protein synthesis is preferably in vitroRNA transcription or in vitroprotein synthesis.

A fifth aspect of the present disclosure provides a cell-free in vitrosynthesis system, comprising: (1) a cell extract; (2) the DNA microsphere according to the first aspect of the present disclosure.

Further preferably, the cell-free in vitrosynthesis system is a cell-free protein synthesis system.

Further preferably, the cell extract is derived from a eukaryotic cell or a prokaryotic cell.

Further preferably, the source of the eukaryotic cell includes, but is not limited to: a mammalian cell, a plant cell, a yeast cell, an insect cell, a nematode cell, and a combination thereof; the source of the mammalian cell may include, but is not limited to, murine origin (including rat, mouse, guinea pig, golden hamster, hamster, etc.), rabbit origin, monkey origin, human origin, pig origin, sheep origin, bovine origin, dog origin, horse origin, etc.

Further preferably, the yeast cell is selected from any one of or a combination of Pichia *pastoris*, Pichia *finlandica*, Pichia *trehalophila*, Pichia *koclamae,* Pichia *membranaefaciens,* Pichia *minuta, Ogataeaminuta*, Pichia *lindneri*, Pichia *opuntiae*, Pichia *thermotolerans*, Pichia *salictaria*, Pichia *guercuum,* Pichia *pijperi*, *Pichiastiptis, Pichia methanolica, Pichia sp ., Saccharomyces cerevisiae, Brewer's yeast, Molasses yeast, Saccharomyces sp., Hansenulapolymorpha, Candida utilis, and Kluyveromyces* sp.

Further preferably, the Kluyveromyces sp. further comprises: the Kluyveromyces sp.comprises: any one of or a combination of Kluyveromyces lactis *(K.lactis*), *Kluyveromyces marxianus, Kluyveromyces dobzhanskii, Kluyveromyces aestuarii, Kluyveromyces nonfermentans, Kluyveromyces wickerhamii, Kluyveromyces thermotolerans, Kluyveromyces fragilis, Kluyveromyces hubeiensis, Kluyveromyces* polysporus, Kluyveromyces siamensis, and Kluyveromyces yarrowii.

A sixth aspect of the present disclosure provides a method for cell-free in vitro synthesis, which is performed using the system provided in the fifth aspect of the present disclosure.

Further preferably, the cell-free in vitrosynthesis method is a cell-free protein synthesis method.

Compared with the prior art, the advantageous effects of the present disclosure are as follows:
(1) High stability: Compared with free DNA template, the DNA template structure linked to the microsphere surface is more stable, degrades more slowly under in vitroreaction conditions, and has a longer reaction lifetime and higher number of uses.
(2) Precise control of template concentration: By controlling the microsphere loading, the concentration of template DNA can be precisely adjusted to meet different reaction requirements.
(3) Low cost: Compared with PCR and gene editing technology, the acquisition cost of microsphere-linked DNA templates is lower, making it easier to achieve industrial production.
(4) Prevention of product contamination: Compared with free DNA template, the linked DNA template can be more easily removed from the reaction product by elution, reducing DNA contamination in the product.

### BRIEF DESCRIPTION OF THE DRAWINGS

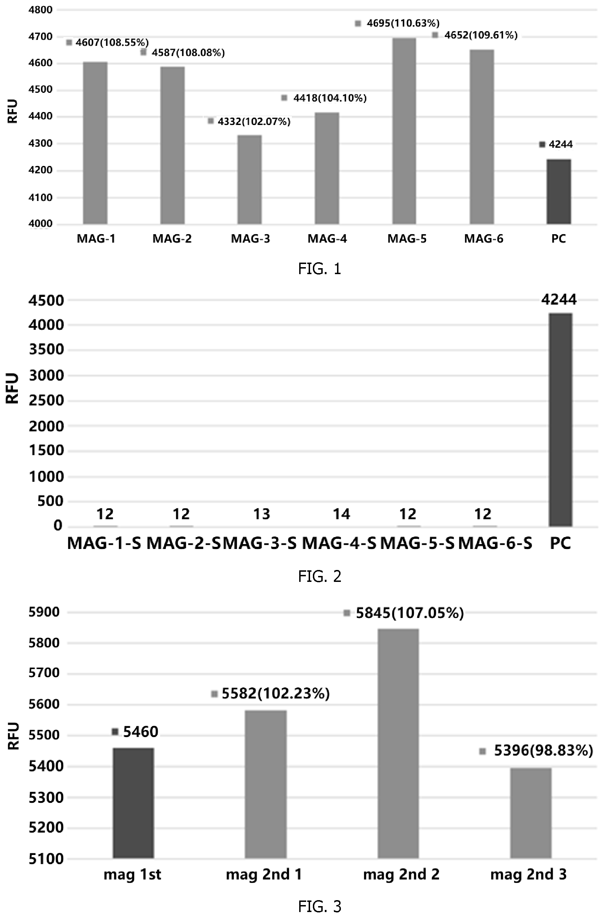

FIG. 1 shows the activity data of the DNA magnetic microspheres prepared in Examples 1-3 of the present disclosure after IVTT reaction, wherein 75 µL of magnetic beads were subjected to activity test by reacting with 200 µL of DNA treated with activation solution at room temperature (15°C), wherein the groups MAG-1 to MAG-6 employed identical pretreatment methods and reaction conditions to verify the reproducibility and degree of stability of the reaction activity. PC represents the activity of DNA not linked to magnetic microspheres.
FIG. 2 shows the activity data of the supernatants corresponding to the DNA magnetic microspheres prepared in Examples 1-3 of the present disclosure after IVTT reaction, wherein the groups MAG-1-S to MAG-6-S employed identical pretreatment methods and reaction conditions to verify the reproducibility and degree of stability of the reaction activity. PC represents the activity of DNA not linked to magnetic microspheres.
FIG. 3 shows the activity comparison between the magnetic microspheres after repeated use and initial use, serving as validation for the effectiveness of the DNA reusable template. mag 1st refers to the activity of the DNA magnetic microspheres during initial use, mag 2nd refers to the activity of the DNA magnetic microspheres after washing (i.e., during the second use), and 1, 2, and 3 refer to three parallel experiments.

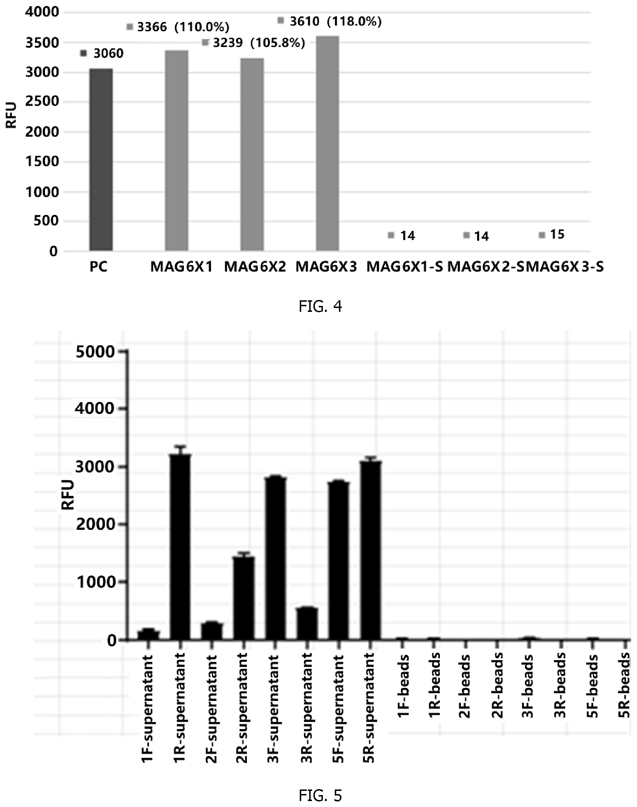

FIG. 4 shows the 6-fold scale-up reaction activity validation of magnetic microspheres, wherein MAG 6X represents 1 µm magnetic microspheres scaled up 6-fold proportionally, 1, 2, and 3 are parallel experiments with identical conditions, -S represents the supernatant of the corresponding group, and PC is the control. (The "scaled up 6-fold proportionally" refers to a 6-fold increase in the amounts of magnetic microspheres, activation solution, and DNA added, representing an overall scale-up of the experiment, while the particle size and concentration of the magnetic microspheres themselves remain unchanged).
FIG. 5 shows the activity data of the DNA magnetic microsphere prepared in Example 5 of the present application and its supernatant after IVTT reaction, wherein F refers to the forward primer (GGATAGCGA, 5' end C6NH2), and R refers to the reverse primer (GCGGTGGCA, 5' end C6NH2). As shown in the figure, 1F-supernatant refers to the first validation experiment of the supernatant of the DNA magnetic microsphere obtained with the forward primer, and 1F-beads refers to the first validation experiment of the DNA magnetic microsphere obtained with the forward primer. 1R-supernatant refers to the first validation experiment of the DNA magnetic microsphere supernatant obtained with the reverse primer, and 1R-beads refers to the first validation experiment of the DNA magnetic microspheres obtained with the reverse primer. The numbers in the figure represent the serial numbers of parallel reactions conducted under identical pretreatment methods and reaction conditions.

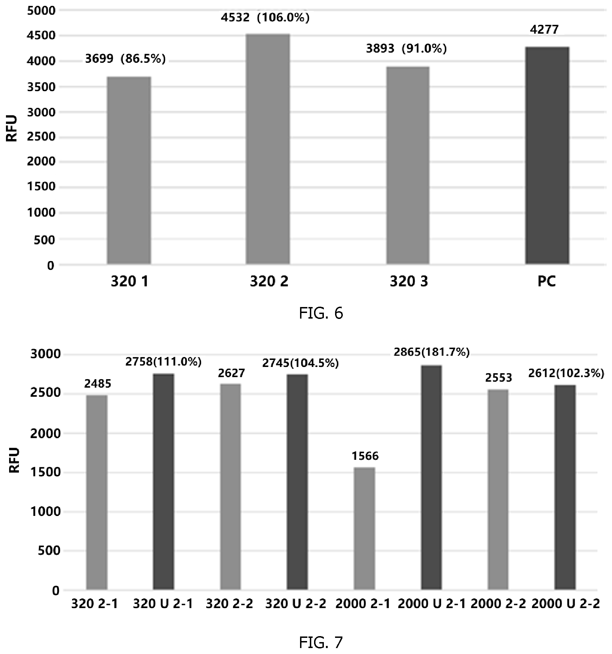

FIG. 6 shows the activity data of the DNA microspheres prepared in Examples 8-10 of the present application after IVTT reaction, wherein 100 µL of glass beads were subjected to activity test by reacting with 200 µL of DNA treated with activation solution at room temperature (15°C), wherein 320 represents the mesh size (45 µm) of the glass beads, and groups 1, 2, and 3 adopted identical pretreatment methods and reaction conditions to verify the reproducibility and degree of stability of the reaction activity. PC represents the activity of DNA not linked to glass microspheres.
FIG. 7 shows the activity data of the supernatant corresponding to the DNA microspheres prepared in Example 12 of the present disclosure after IVTT reaction, which is used to verify the effect of ultrasonic concussion on the activity of glass beads, wherein 320 and 2000 represent the mesh size of the glass beads (corresponding to particle sizes of 45 µm and 7.5 µm, respectively), and -1 and -2 indicate that the identical pretreatment methods and reaction conditions were adopted, aiming to verify the reproducibility and degree of stability of the reaction activity. U indicates that the corresponding group was subjected to ultrasonic concussion for 10 min before the reaction.

### DETAILED DESCRIPTION OF THE PREFERRED EMBODIMENT

The present disclosure is further illustrated below in conjunction with specific examples and examples. Please refer to the accompanying drawings. It should be understood that these examples are only used to illustrate the present disclosure and not to limit the scope of the present disclosure. For experimental methods without specified conditions in the following examples, priority is given to the conditions described in the above specific implementation guidance, and then conventional conditions may be followed, such as "Sambrook et al., Molecular Cloning: Laboratory Manual (New York: Cold Spring Harbor Laboratory Press, 1989)", "Edited by Alexander S. Spirin and James R. Swartz. Cell-free protein synthesis: methods and protocols[M]. 2008" and other literature, or according to the conditions recommended by the manufacturer.

Unless otherwise specified, the percentages and parts mentioned in the present disclosure are weight percentage and parts by weight.

Unless otherwise specified, the materials and reagents used in the examples of the present disclosure are commercially available products.

Unless otherwise specified, the temperature units in this application are in degrees Celsius (°C).

### Terms and Definitions

The following provides explanations or clarifications regarding the meanings of certain "terms" and "definitions" used in the present disclosure to facilitate better understanding. The corresponding explanations or descriptions apply to the entire text of the present disclosure, applicable both to the following and the preceding sections. When references to literature are involved in the present disclosure, the definitions of related terms, nouns, and phrases in the referenced literature are also incorporated by reference. However, in case of conflict with the definitions in the present disclosure, the definitions in the present disclosure shall prevail. In case of any conflict between the definitions in the cited references and those in the present disclosure, it shall not affect the referenced components, substances, compositions, materials, systems, formulations, species, methods, equipment, etc., which shall be determined based on the content specified in the cited references.

In the description of the present disclosure, preferred embodiments described by expressions such as "one of the preferred", "one of the preferred modes", "one of the preferred embodiments", "one of the preferred examples", "preferred examples", "in a preferred embodiment", "in some preferred examples", "in some preferred modes", "preferably is", "preferably", "preferable", "more preferably", "more preferable", "further preferably", "most preferably", as well as illustrative enumerations described by expressions such as "one of the embodiments", "one of the modes", "example", "specific example", "for example", "as an example", "e.g.", "for instance", "such as", and other, shall not in any sense limit the scope of coverage or protection of the invention. The specific features described by the various expressions are included in at least one specific embodiment of the present disclosure. In the present disclosure, the specific features described by the various expressions may be combined in any suitable manner in one or more specific embodiments. In the present disclosure, the technical features or technical solutions corresponding to the preferred embodiments may also be combined in any suitable manner.

In the present disclosure, "any combination thereof" quantitatively means "greater than 1" and in terms of coverage scope means a group consisting of the following scenarios: "optionally one of", or "optionally a group consisting of at least two of".

In the present disclosure, the expressions "one or more", "one or a plurality of", and similar "one or multiple" have the same meaning as "at least one", "at least one kind", "a combination of", "or a combination of", "and a combination of", "or any combination of", "and any combination of", and the like, and may be used interchangeably to indicate a quantity equal to "1" or "greater than 1".

In the present disclosure, the use of "or/and", "and/or" indicates "either optionally one or any combination of", and also means at least one of.

The terms "typically", "conventionally", "generally", "frequently", and "often" used in the present disclosure to describe prior art methods are also cited as references for the content of the present disclosure. Unless otherwise specified, they may be considered as one of the preferred embodiments for some technical features of the present disclosure. It should be noted that these terms do not in any sense limit the coverage or scope of protection of the invention.

All documents mentioned in the present disclosure, as well as documents directly or indirectly cited by these documents, are incorporated herein by reference as if each document were individually incorporated by reference.

It should be understood that within the scope of the present disclosure, the aforementioned technical features of the present disclosure and the various technical features specifically described below (including but not limited to the examples) can be mutually combined to form new or preferred technical solutions, as long as they can be used to implement the present disclosure. Due to space limitations, they will not be exhaustively listed.

Polymer, which is broadly defined in the present disclosure to include oligomers and high polymers, having at least three structural units or a molecular weight of at least 500 Da (the molecular weight may be determined by suitable characterization methods, such as number average molecular weight, weight average molecular weight, viscosity average molecular weight, etc.).

Acrylic monomer molecule: monomer molecules usable for synthesizing acrylic polymers, having the basic structure of C(COO-)=C, exemplified by CH(COOH)=CH₂, CH(COONa)=CH₂, CH₃C(COOH)=CH₂, CH₃C(COONa)=CH₂, CH(COOCH₃)=CH₂, CH(COOCH₂CH₂OH)=CH₂, CH₃C(COOCH₃)=CH₂, CH₃C(COOCH₂CH₂OH)=CH₂, and the like.

Fixed, fixed to, fixed with, fixed on, and other "fixed" modes refer to covalent bonding modes.

The terms "with", "connected to", "linked to", "attached to", "bound", "captured", "trapped", and other "connection"/"binding" modes are not particularly limited and include, but are not limited to, covalent and non-covalent modes.

Covalent mode: direct bonding mode via covalent bond. The covalent mode includes, but is not limited to, dynamic covalent mode, wherein the dynamic covalent mode refers to direct bonding mode via dynamic covalent bond.

Covalent bond: including common covalent bonds such as amide bond and ester bond, as well as dynamic covalent bond with reversible properties.

The covalent bond includes a dynamic covalent bond. A dynamic covalent bond is a type of chemical bond with reversible properties, including but not limited to imine bonds, acylhydrazone bonds, disulfide bonds, or combinations thereof. Those skilled in the chemical art will understand the meaning of covalent bond.

"Modified" products, including but not limited to derivatives, modified products, genetically engineered products, fusion products, etc. of the present disclosure, may retain their original functions or properties, or may optimize or alter their functions or properties.

In vitro protein synthesis reaction refers to a reaction for synthesizing proteins in a cell-free synthesis system in vitro, includes at least the translation process, and includes but not limited to IVT reaction (in vitrotranscription reaction), IVTT reaction (in vitrotranscription-translation reaction), and IVDTT reaction (in vitroreplication-transcription-translation reaction). In the present disclosure, the IVTT reaction is preferred. The IVTT reaction, corresponding to the IVTT system, is a process of transcribing and translating DNA into protein in *vitro*. Therefore, such in vitroprotein synthesis systems are also referred to as D2P systems, D-to-P systems, D_to_P systems, or DNA-to-Protein systems. The corresponding in vitroprotein synthesis method, also referred to as the D2P method, D-to-P method, D_to_P method, or DNA-to-Protein method.

The "cell-free system" refers to an in vitrobiosynthesis approach that does not rely on secretion and expression by intact cells. It should be noted that the in vitrosynthesis system of the present disclosure also allows the addition of cellular components to facilitate the reaction, but the added cells are not primarily intended for secretory expression of exogenous target proteins.

Furthermore, circumvention attempts involving intentional addition of a small amount of intact cells (for example, the protein content provided by the intact cells does not exceed 30 wt% compared to that provided by the cell extract) in the D2P system devoid of intact cells constructed under the guidance of the present disclosure is also encompassed within the scope of protection of the present disclosure.

Exogenous protein: The expression product of interest of the in vitroprotein synthesis system of the present disclosure is not synthesized and secreted by the host cell, but is synthesized *in* vitrobased on the exogenous nucleic acid template, and thus may also be referred to as the target protein. The exogenous protein may be a protein, a fusion protein, a mixture containing protein molecules or fusion protein molecules; it also broadly includes polypeptides. The product obtained after performing an in vitro protein synthesis reaction based on a nucleic acid template encoding an exogenous protein may be a single substance or a combination of two or more substances. The terms "exogenous protein", "protein of interest", "target protein", and "target translation product" have the same meaning and may be also referred to as "objective protein", "interested protein", "objective translated product", or "interested protein product", and are used interchangeably in the present disclosure.

D2P is an abbreviation for DNA-to-Protein, indicating the process from a DNA template to a protein product. For example, D2P technology, D2P system, D2P method, D2P kit, etc.

"The expression system of the present disclosure", "the in vitro expression system of the present disclosure", "in vitrocell-free expression system", and "in vitro cell-free expression platform" are used interchangeably, all referring to the in vitro expression system of the present disclosure, and may also be described by other terms such as: protein in vitrosynthesis system, in vitroprotein synthesis system, cell-free system, cell-free platform, cell-free protein synthesis system, cell-free in vitroprotein synthesis system, in vitro cell-free protein synthesis system, in vitrocell-free synthesis system, CFS system (cell-free system), CFPS system (cell-free protein synthesis system), and other descriptions. According to the reaction mechanism, it may include an in vitrotranscription system (abbreviated as IVT system, an mR2P system), an in vitrotranscription-translation system (abbreviated as IVTT system, a D2P system), an in vitroreplication-transcription-translation system (abbreviated as IVDTT system, a D2P system), etc. In the present disclosure, the IVTT system is preferred. We also refer to the in vitroprotein synthesis system as "protein synthesis factory" ("Protein Factory" or "proteinfactory" or "Proteinfactory"). The in vitroprotein synthesis system provided by the present disclosure adopts an open-ended description for its components. The cell-free protein synthesis system of the present disclosure uses exogenous DNA, mRNA, or a combination thereof as the nucleic acid template for protein synthesis, and achieves in vitrosynthesis of the target protein by artificially controlling the supplementation of substrates required for protein synthesis and substances such as transcription- and translation-related protein factors.

One specific embodiment of the in *vitro* protein synthesis system further includes, but is not limited to, for example, the Escherichia coli-based cell-free protein synthesis system described in WO 2016005982 A1. In vitro cell-free protein synthesis systems described in other cited references of the present disclosure, and the direct and indirect cited references thereof-including, but not limited to, in vitrocell-free protein synthesis systems based on wheat germ cells, rabbit reticulocytes, Saccharomyces cerevisiae, Pichia pastoris, and Kluyveromyces marxianus-are also incorporated into the present disclosure as embodiments of the in vitro protein synthesis system of the present disclosure. For example, the literature "Lu, Y. Advances in Cell-Free Biosynthetic Technology. Current Developments in Biotechnology and Bioengineering, 2019, Chapter 2, 23-45" includes, but is not limited to, the in vitro cell-free protein synthesis systems described in the references cited in the "2.1 Systems and Advantages" section on pages 27-28, all of the in vitrocell-free protein synthesis systems can serve as the in vitroprotein synthesis system for implementing the present disclosure. For example (unless in conflict with the present disclosure, the following documents and the documents cited therein are incorporated herein by reference in their entirety and for all purposes), the in vitrocell-free protein synthesis systems, DNA template construction and amplification methods described in CN 106978349 A, CN 108535489 A, CN 108690139 A, CN 108949801 A, CN 108642076 A, CN 109022478 A, CN 109423496 A, CN 109423497 A, CN 109423509 A, CN 109837293 A, CN 109971783 A, CN 109988801 A, CN 109971775 A, CN 110093284 A, CN 110408635 A, CN 110408636 A, CN 110551745 A, CN 110551700 A, CN 110551785 A, CN 110819647 A, CN 110845622, CN 110938649 A, CN 110964736 A, CN 111378706 A, CN 111378707 A, CN 111378708 A, CN 111718419 A, CN 111748569 A, CN 2019107298813, CN 2019112066163, CN 2018112862093, CN 2019114181518, CN 2020100693833, CN 2020101796894, CN 202010269333X, CN 2020102693382, CN 2020113574616 and the documents cited therein can all serve as the in vitroprotein synthesis system of the present disclosure, and the DNA template construction and amplification methods of the present disclosure.

In the present disclosure, "protein" and "proteins" have the same meaning and can be used interchangeably.

In the present disclosure, the terms "system" and "systems" can be used interchangeably.

In the present disclosure, "protein synthesis amount", "protein expression level", and "protein expression yield" have the same meaning and can be used interchangeably.

In the present disclosure, the terms "cell extract", "cell extract liquid", "cell lysate", "cell disruption product", and "cell lysate product" have the same meaning and can be used interchangeably.

In the present disclosure, the terms "energy system", "energy systems", and "energy supply system" have equivalent meanings and can be used interchangeably. The energy regeneration system has the same meaning as the energy regeneration systems and can be used interchangeably. The energy regeneration system is a preferred embodiment or component of the energy system.

The in vitrosynthesis system of the present disclosure further comprises one or more components selected from the following group: a substrate for protein synthesis, a substrate for RNA synthesis, RNA polymerase, magnesium ions, potassium ions, a buffering agent, an energy regeneration system, polyethylene glycol (PEG) or analogs thereof, dithiothreitol (DTT), and optionally a solvent, wherein the solvent is water or an aqueous solvent. Further, the cell extract does not contain yeast endogenous long-chain nucleic acid molecules.

Further, the substrate for RNA synthesis comprises: nucleoside monophosphate, nucleoside triphosphate, or a combination thereof.

Further, the substrate for protein synthesis comprises: 20 natural amino acids and unnatural amino acids.

Further, the magnesium ions are derived from a magnesium ion source, wherein the magnesium ion source is selected from the following group: one of or a combination of magnesium acetate and magnesium glutamate.

Further, the potassium ions are derived from a potassium ion source, wherein the potassium ion source is selected from the following group: one of or a combination of potassium acetate and potassium glutamate.

Further, the energy regeneration system is selected from the following group: one of creatine phosphate/phosphocreatinase system and glycolysis intermediate product energy system, sucrose, or a combination thereof.

Further, the buffering agent is selected from the following group: 4-(2-hydroxyethyl)piperazine-1-ethanesulfonic acid, tris(hydroxymethyl)aminomethane, or a combination thereof.

Further, the protein synthesis system contains polyethylene glycol (PEG) or analogs thereof. The concentration of polyethylene glycol or analogs thereof is not particularly limited. Generally, the concentration (w/v) of polyethylene glycol or analogs thereof is 0.1-8%, preferably 0.5-4%, more preferably 1-2%, based on the total weight of the protein synthesis system. Representative PEGs are selected from the following group: PEG3000, PEG3350, PEG6000, PEG8000, or a combination thereof.

Further, the polyethylene glycol comprises polyethylene glycol with a molecular weight (Da) of 200-10000, such as PEG200, 400, 1500, 2000, 4000, 6000, 8000, 10000, etc., preferably, polyethylene glycol with a molecular weight of 3000-10000.

Optionally, the protein synthesis system provided by the present disclosure comprises: yeast cell extract, 4-(2-hydroxyethyl)piperazine-1-ethanesulfonic acid, potassium acetate, magnesium acetate, adenosine triphosphate (ATP), guanosine triphosphate (GTP), cytidine triphosphate (CTP), thymidine triphosphate (TTP), amino acid mixture, creatine phosphate, dithiothreitol (DTT), creatine phosphate kinase, RNA polymerase, polyethylene glycol, and sucrose.

In the present disclosure, the cell extract does not contain intact cells, and typical cell extracts include ribosomes, transfer RNA, and aminoacyl-tRNA synthetase for protein translation, initiation factors and elongation factors required for protein synthesis, as well as termination release factors. In addition, the cell extract further contains other proteins derived from the cytoplasm of the cells, particularly soluble proteins.

In the present disclosure, the protein content in the cell extract is 20-100 mg/mL, preferably 50-100 mg/mL. The method for determining the protein content is the Coomassie Brilliant Blue assay.

In the present disclosure, the preparation method of the cell extract or cell lysate is not limited, and a preferred preparation method comprises the following steps:
(i) providing cells;
(ii) subjecting the cells to a washing treatment to obtain washed cells;
(iii) subjecting the washed cells to cell disruption to obtain a cell crude extract;
(iv) subjecting the cell crude extract to solid-liquid separation to obtain a liquid portion, which is the cell extract.

In the present disclosure, the solid-liquid separation method is not particularly limited, and a preferred method is centrifugation.

In the present disclosure, the condition for the centrifugation is not particularly limited, and a preferred centrifugation condition is 5000-100000×g, more preferably, 8000-30000×g.

In the present disclosure, the duration of the centrifugation is not particularly limited, and a preferred duration of centrifugation is 0.5 min-2 h, more preferably, 20 min-50 min.

In the present disclosure, the temperature for the centrifugation is not particularly limited, and preferably, the centrifugation is performed at 1-10°C, more preferably at 2-6°C.

In the present disclosure, the method for the washing treatment is not particularly limited. A preferred method for the washing treatment involves using a washing solution for treatment at a pH of 7-8 (more preferably 7.4). The washing solution is not particularly limited, and typical washing solutions are selected from the following group: potassium 4-(2-hydroxyethyl)piperazine-1-ethanesulfonate, potassium acetate, magnesium acetate, or a combination thereof.

In the present disclosure, the method for cell disruption is not particularly limited, and a preferred cell disruption treatment comprises high-pressure disruption, freeze-thaw (e.g., liquid nitrogen cryogenic) disruption.

The nucleoside triphosphate mixture in the protein synthesis system is adenosine triphosphate, guanosine triphosphate, cytidine triphosphate, and uridine triphosphate. In the present disclosure, the concentration of each mononucleotide is not particularly limited, and is generally 0.5-5 mM for each mononucleotide, preferably 1.0-2.0 mM.

The amino acid mixture in the protein synthesis system may include natural or unnatural amino acids, and may include D- or L-amino acids. Representative amino acids include (but are not limited to) 20 natural amino acids: glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, and histidine. The concentration of each amino acid is typically 0.01-0.5 mM, preferably 0.02-0.2 mM, such as 0.05, 0.06, 0.07, 0.08 mM.

In a preferred embodiment, the in vitrocell-free protein synthesis system further comprises sucrose, wherein the concentration of the sucrose is 0.03-40 wt%, preferably 0.08-10 wt%, more preferably 0.1-5 wt%, based on the total weight of the protein synthesis system.

A particularly preferred in vitrocell-free protein synthesis system, in addition to yeast cell extract, further comprises the following components: 22 mM 4-(2-hydroxyethyl)piperazine-1-ethanesulfonic acid at pH 7.4, 30-150 mM potassium acetate, 1.0-5.0 mM magnesium acetate, 1.5-4 mM nucleoside triphosphate mixture, 0.08-0.24 mM amino acid mixture, 25 mM creatine phosphate, 1.7 mM dithiothreitol, 0.27 mg/mL creatine phosphate kinase, 1%-4% polyethylene glycol, 0.5%-2% sucrose, 0.027-0.054 mg/mL T7 RNA polymerase.

### Cell extract

In the present disclosure, the terms "cell extract" and "cell lysate" convey identical meanings, both referring to the material obtained after cell disruption.

### Example 1: Modification of magnetic microspheres

1 to 150 g of silica-coated ferroferric oxide magnetic microspheres were weighed, and washed with anhydrous ethanol 2 to 5 times; 5 to 500 mL of an ethanol solution of 3-aminopropyltriethoxysilane (APTES, CAS: 919-30-2) (5% to 20%, v/v) was added to the washed magnetic microspheres, reacted for 12 to 72 hours, then the magnetic microspheres was washed with anhydrous ethanol and distilled water 3 to 5 times to obtain amino-modified magnetic microspheres A.

### Example 2: Preparation of DNA sample

(1) Preparation of polyacrylic acid solution a
   a. 4 mL of polyacrylic acid solution (molecular weight 3000, mass fraction 50%) was taken, ddH₂O was added to make up to 40 mL, the pH was adjusted to 7.5 by dropwise addition of 1 mol/L NaOH, and then pure water was added to bring the volume to 50 mL.
   b. 230 µL of the solution was transferred to a test tube, ddH₂O was added to make up to 10 mL, and the mixture was shaken thoroughly.
(2) 318 µL of polyacrylic acid solution was added to the DNA primer (specific sequence: GGCGTAGAGGAT, with amino modification at C6 position of the 5' end during synthesis), mixed thoroughly using a vortex mixer for 5 min, and the mixture was allowed to stand for 25 min.
(3) the modified DNA primer sequence (polyacrylic acid-modified DNA primer sequence) was used for amplification to obtain the green fluorescent protein DNA sample (DNA template).

### Example 3: Preparation of DNA magnetic microspheres.

(1) 75 µL of magnetic microsphere A (particle size: 1 µm, containing liquid) was transferred to a 2 mL centrifuge tube a.
(2) 200 µL of DNA sample was transferred to another centrifuge tube b.
(3) the activation solution was prepared by weighing 0.300 g of EDC and 0.150 g of NHS into a 15 mL centrifuge tube, ddH₂O was added to make up to 10 mL, and the mixture was mixed thoroughly to ensure complete dissolution of the reagents.
(4) 100 µL of the activation solution was transferred to the centrifuge tube b, mixed thoroughly using a vortex mixer for 1 min, and the mixture was allowed to stand for 15 min.
(5) the entire mixed solution in the centrifuge tube b was transferred to the centrifuge tube a, the centrifuge tube a was manually shaken to ensure thorough mixing of the contents in the tube.
(6) the centrifuge tube a was placed on a rotator for reaction at room temperature (preferably 18°C) for 24 h to obtain a solution containing DNA magnetic microspheres.

### Example 4: Activity validation

(1) the centrifuge tube a was removed and placed on a magnetic test tube rack, and allowed to stand for separation of DNA magnetic microspheres from the liquid.
(2) the supernatant was transferred to a centrifuge tube c.
(3) ddH₂O was added to the centrifuge tube a for washing, then the supernatant was completely aspirated.
(4) the centrifuge tube a and the centrifuge tube c were submitted for testing, the target protein was synthesized in the IVTT system.

The IVTT system comprises the following components (final concentrations): 9.78 mM Tris-HCl at pH 8.0, 80 mM potassium acetate, 5 mM magnesium acetate, 1.8 mM nucleoside triphosphate mixture (adenosine triphosphate, guanosine triphosphate, cytidine triphosphate, and uridine triphosphate, each at a concentration of 1.8 mM), 0.7 mM amino acid mixture (glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, and histidine, each at a concentration of 0.1 mM), 15 mM glucose, 320 mM maltodextrin (calculated as glucose units in molar concentration, corresponding to approximately 52 mg/mL), 24 mM tripotassium phosphate, 2% polyethylene glycol 8000, and finally 50% volume of cell extract (specifically yeast cell extract, more specifically Kluyveromyces lactis cell extract).

The Kluyveromyces lactisextract comprises endogenously expressed T7 RNA polymerase. The Kluyveromyces lactis extract is modified by the following means: An engineered strain based on Kluyveromyces lactis strain ATCC8585 was employed; following the method described in CN 109423496 A, the coding gene of T7 RNA polymerase was integrated into the genome of Kluyveromyces *lactis* to obtain the engineered strain, enabling endogenous expression of T7 RNA polymerase in the engineered strain; a cell material was cultured from the engineered strain, and then the cell extract was prepared. The preparation process of Kluyveromyces lactisyeast cell extract employed conventional technical means, with reference to the method described in CN 109593656 A. The preparation steps are summarized as follows: an appropriate amount of Kluyveromyces lactis cells from fermentation culture was provided, the cells were rapidly froze with liquid nitrogen, the cells were disrupted, and the supernatant was collected by centrifugation to obtain the cell extract. The protein concentration in the obtained Kluyveromyces lactiscell extract was 20-40 mg/mL.

IVTT reaction: the components in the centrifuge tube a and the centrifuge tube c were added to the in vitroprotein synthesis system to perform the in vitro protein synthesis reaction. The protein was synthesized to obtain an IVTT reaction solution containing the protein. Fluorescent protein activity assay: the reaction solution was placed in an Envision 2120 multimode plate reader (Perkin Elmer) to detect the fluorescence signal intensity, with the relative fluorescence unit (RFU) used as the activity unit (see FIGs. 1-2 for details).

As shown in FIGs. 1-2, the amount of free template DNA in the supernatant is minimal, and the activity is almost negligible, indicating that nearly all template DNA is linked to the magnetic microspheres, and the resulting DNA magnetic microspheres exhibit high biological activity. Additionally, the activity data from MAG-1 to MAG-6 demonstrated that the magnetic bead activities under 6 identical reaction conditions were RFU4607, RFU4587, RFU4332, RFU4418, RFU4695, and RFU4652, respectively, all exceeding those of the control group PC (MAG-3 was the lowest at 102.07% of PC activity, while MAG-5 was the highest at 110.63% of PC activity), with a maximum activity difference of RFU363 (8.38%) among the groups. The activity requirements were met. This indicated that the DNA magnetic microsphere maintains high activity as template DNA even after repeated use.

### Example 5: Repeated testing of DNA magnetic microspheres after washing

The DNA magnetic microspheres from the above examples were washed and then subjected to IVTT activity test again, with parallel tests conducted under the same conditions as in Example 4. The results of 3 parallel tests showed that the magnetic bead activity (RFU5396-RFU5845) after washing and reuse once was 98.83%-107.05% of the initial activity (RFU5460). The maximum decay rate was 1.17%. Assuming the activity decreased by 1.17% with each repetition, the activity should be 79.03% after 20 repetitions. When the PC activity was RFU5460, the magnetic bead activity should be RFU4315, still meeting the activity requirements (refer to FIG. 3).

### Example 6: Scale-up experimental verification

The amounts of magnetic microsphere A, activation solution, and DNA were scaled up by 6 times, and the above experiment was repeated for verification. As shown in FIG. 4, the activities of MAG 6X 1, 2, and 3 groups were RFU3366, RFU3239, and RFU3610, respectively, all above 100% of the PC control group, with the highest being MAG 6X 3 at 118% of PC activity and the lowest being MAG 6X 2 at 105.8% of PC activity. This proved that even when the reaction conditions were scaled up several times, the DNA sequences were still almost entirely linked to the microspheres, and the DNA magnetic microsphere maintains high activity.

### Example 7 (comparative example): The DNA primer sequence was linked to the magnetic microsphere followed by amplification, and the activity of the magnetic microsphere was verified.

(1) 75 µL of magnetic microsphere A (particle size: 1 µm, containing liquid) was transferred to a 2 mL centrifuge tube a.
(2) 200 µL of the amino-modified DNA primer sequence was transferred to another centrifuge tube b.
(3) the activation solution was prepared by weighing 0.300 g of EDC and 0.150 g of NHS into a 15 mL centrifuge tube, ddH₂O was added to make up to 10 mL, and the mixture was mixed thoroughly to ensure complete dissolution of the reagents.
(4) 100 µL of the activation solution was transferred to the centrifuge tube b, mixed thoroughly using a vortex mixer for 1 min, and the mixture was allowed to stand for 15 min.
(5) the entire mixed solution in the centrifuge tube b was transferred to the centrifuge tube a, the centrifuge tube a was manually shaken to ensure thorough mixing of the contents in the tube.
(6) the centrifuge tube a was placed on a rotator for reaction at room temperature for 24 h.
(7) the centrifuge tube a was removed and placed on a magnetic test tube rack, and allowed to stand for separation of the magnetic beads from the liquid.
(8) the supernatant was taken and transferred to a centrifuge tube c.
(9) ddH₂O was added to the centrifuge tube a for washing 3 times, then the supernatant was completely aspirated.
(10) the magnetic beads linked to DNA sequences in the centrifuge tube a and the free DNA sequences in centrifuge tube c were submitted for amplification (green fluorescent protein DNA template).
(11) the fluorescent protein activity assay was performed in samples from centrifuge tubes a and c after amplification (method same as in Example 4): the reaction solution was placed in an Envision 2120 multimode plate reader (Perkin Elmer) to detect the fluorescence signal intensity, with the relative fluorescence unit (RFU) used as the activity unit (as shown in FIG. 5).

As shown in FIG. (5), after linking the DNA sequences with magnetic beads and performing the amplification reaction, the magnetic beads were washed post-amplification and subjected to an IVTT reaction in comparison with the supernatant. The washed magnetic beads exhibited almost no activity, while the supernatant showed relatively high activity, indicating that the DNA primer sequence, after linked to the magnetic beads and subsequent amplification, had extremely low activity. This demonstrated that the DNA magnetic beads prepared using the method of the present disclosure exhibited high activity.

### Example 8: Microsphere modification

1-50 g of glass microspheres were weighed, ultrasonically washed 1-3 times with distilled water, and 5-250 mL of 1-2 mol/L NaOH solution was added to the washed glass microspheres. The reaction was carried out at 70°C for 1-2 h, followed by washing 5-10 times with distilled water. Then, 5-200 mL of an ethanol solution of 3-aminopropyltriethoxysilane (APTES, CAS: 919-30-2) (5%-20%, v/v) was added to the washed glass microspheres, and the reaction was carried out for 12-72 h. Finally, the glass microspheres were washed 3-5 times with anhydrous ethanol and distilled water to obtain modified glass microsphere A.

### Example 9: Preparation of DNA sample

(1) Preparation of polyacrylic acid solution a
   a. 4 mL of polyacrylic acid solution (molecular weight 3000, mass fraction 50%) was taken, ddH₂O was added to make up to 40 mL, the pH was adjusted to 7.5 by dropwise addition of 1 mol/L NaOH, and then pure water was added to bring the volume to 50 mL.
   b. 230 µL of the solution was transferred to a test tube, ddH₂O was added to make up to 10 mL, and the mixture was shaken thoroughly.
(2) 318 µL of polyacrylic acid solution was added to the DNA primer sequence (the experiment used artificially synthesized DNA having the specific sequence GGCGTAGAGGAT, with amino modification at C6 position of the 5' end during synthesis), mixed thoroughly using a vortex mixer for 5 min, and the mixture was allowed to stand for 25 min.
(3) the modified DNA primer sequence was used for amplification (Ampi) to obtain the green fluorescent protein DNA sample.

### Example 10: Preparation of DNA glass microspheres.

(1) Preparation of MES-KOH buffer
   ① 1.952 g of MES was taken and placed into a beaker;
   ② approximately 60 mL of ddH₂O was added to the beaker, and stirred to dissolve MES.
   ③ 1 mol/L KOH solution was added dropwise to the beaker to adjust the pH to 5.6.
   ④ the liquid in the beaker was transferred to a 100 mL volumetric flask and diluted to volume.
(2) glass microsphere A (320 mesh, 45 µm) was washed until the pH drops to 7-8.
(3) 100 µL of the washed glass microsphere A (without liquid) was transferred to a centrifuge tube a, washed 3 times with MES-KOH buffer, and centrifuged to remove the supernatant.
(4) 100 µL of MES-KOH buffer was added to the centrifuge tube a.
(5) 200 µL of green fluorescent protein DNA sample was taken and transferred to another centrifuge tube b.
(6) the activation solution was prepared by weighing 0.300 g of EDC and 0.150 g of NHS into a 15 mL centrifuge tube, MES-KOH buffer was added to make up to 10 mL, and the mixture was mixed thoroughly to ensure complete dissolution of the reagents.
(7) 100 uL of the activation solution was transferred to the centrifuge tube b, mixed thoroughly using a vortex mixer for 1 min, and the mixture was allowed to stand for 15 min.
(8) the entire mixed solution in the centrifuge tube b was transferred to the centrifuge tube a, the centrifuge tube a was manually shaken to ensure thorough mixing of the contents in the tube.
(9) the centrifuge tube a was placed on a rotator for reaction at room temperature (preferably 15°C) for 24 h.

### Example 11: Activity validation

(1) the centrifuge tube a was removed and placed on a test tube rack, and allowed to stand for separation of microspheres from the liquid.
(2) the supernatant was transferred to a centrifuge tube c.
(3) ddH₂O was added to the centrifuge tube a for washing 3 times, then the supernatant was completely aspirated.
(4) the centrifuge tube a and the centrifuge tube c were submitted for testing, the target protein was synthesized in the IVTT system (same as in Example 4).

As shown in FIG. 6, three parallel experiments were conducted on 320-mesh microspheres. The activities of DNA microspheres in groups 1, 2, and 3 were determined to be RFU3699, RFU4532, and RFU3893, respectively. The highest activity was observed in group 2, reaching 106% of PC activity. Even the lowest activity in group 1 reached 86.5% of PC activity. It could be seen that the DNA glass microsphere of the present disclosure as a DNA template also exhibited good reaction activity, along with favorable reproducibility and stability.

### Example 12: Verification of the effect of ultrasonic concussion on the activity of DNA microspheres.

The steps were similar to those in Example 10, except that step (4-1) was added between steps (4) and (5) of Example 10: That is, the glass microspheres resuspended in buffer solution after washing were placed in an ultrasonic processor and subjected to ultrasonic concussion for 10 min, with other steps referring to Example 10. Subsequently, activity validation of the DNA microspheres was performed according to the method of Example 11, while the impact of ultrasonic concussion on the activity of DNA microspheres with different particle sizes was also validated. Multiple parallel experiments were conducted for effective validation (see FIG. 7 for details).

As shown in FIG. 7, the DNA microspheres subjected to ultrasonic treatment followed by linkage, whether 320-mesh or 2000-mesh (7.5 µm), exhibited higher activity than the corresponding DNA microspheres without ultrasonic concussion treatment, and this conclusion remained valid after repeated experimental verification. The group with the highest improvement was 2000 U2-1, showing an 81.7% increase in RFU, while the lowest was 2000 U2-2, with a 2.3% increase in RFU. The above experiments demonstrated that subjecting the glass microsphere suspension to ultrasonic concussion prior to linking the glass microspheres to DNA could significantly enhance the reaction activity of the DNA microspheres. This method was applicable to microspheres of different particle sizes while maintaining good repeatability and stability.

Based on the ideal examples of the present application as inspiration, through the above description, relevant personnel can make various changes and modifications without departing from the technical concept of this application. The technical scope of this application is not limited to the content of the specification and must be determined based on the scope of the claims.

## Claims

1. A DNA microsphere, comprising: a microsphere body, a polymer connected to the surface of the microsphere body, and DNA bound to the polymer.

2. The DNA microsphere according to claim 1, wherein the polymer binds to DNA via a functional group; the functional group is selected from a carboxyl group, a hydroxyl group, an amino group, a sulfhydryl group, a salt form of a carboxyl group, a salt form of an amino group, an ester group, or a combination of the foregoing functional groups.

3. The DNA microsphere according to claim 1 or 2, wherein the DNA is modified by the functional group.

4. The DNA microsphere according to any one of claims 1-3, wherein the polymer is directly connected or indirectly connected to the outer surface of the microsphere body via a linking group.

5. The DNA microsphere according to claim 4, wherein the linking group is selected from one or a combination of substituted or unsubstituted siloxy group, amide group, ester group, carbonyl group, alkenyl group, alkynyl group, alkylene group, ether group, and thioether group.

6. The DNA microsphere according to any one of claims 1-5, wherein the surface of the microsphere body contains silicon or silicon oxide; preferably, the material of the microsphere body is selected from a glass material, a ceramic material, or a magnetic material, and when the microsphere body is a magnetic microsphere, the surface of the microsphere body is coated with silicon dioxide.

7. The DNA microsphere according to any one of claims 1-6, wherein the size of the microsphere body is selected from any one of the following particle size scales or a range between any two particle size scales: 0.1 µm, 0.15 µm, 0.2 µm, 0.25 µm, 0.3 µm, 0.35 µm, 0.4 µm, 0.45 µm, 0.5 µm, 0.55 µm, 0.6 µm, 0.65 µm, 0.7 µm, 0.75 µm, 0.8 µm, 0.85 µm, 0.9 µm, 0.95 µm, 1 µm, 1.5 µm, 2 µm, 2.5 µm, 3 µm, 3.5 µm, 4 µm, 4.5 µm, 5 µm, 5.5 µm, 6 µm, 6.5 µm, 7 µm, 7.5 µm, 8 µm, 8.5 µm, 9 µm, 9.5 µm, 10 µm, 15 µm, 20 µm, 25 µm, 30 µm, 35 µm, 40 µm, 45 µm, 50 µm, 55 µm, 60 µm, 65 µm, 70 µm, 75 µm, 80 µm, 85 µm, 90 µm, 95 µm, 100 µm, 150 µm, 200 µm, 250 µm, 300 µm, 350 µm, 400 µm, 450 µm, 500 µm, 550 µm, 600 µm, 650 µm, 700 µm, 750 µm, 800 µm, 850 µm, 900 µm, 950 µm, and 1000 µm; the diameter dimensions are average values;
preferably, the diameter of the microsphere body is selected from 0.1-10 µm;
preferably, the diameter of the microsphere body is selected from 0.2-6 µm;
preferably, the diameter of the microsphere body is selected from 0.4-5 µm;
preferably, the diameter of the microsphere body is selected from 0.5-3 µm;
preferably, the diameter of the microsphere body is selected from 0.2-1 µm;
preferably, the diameter of the microsphere body is selected from 0.5-1 µm;
preferably, the diameter of the microsphere body is selected from 1 µm-1 mm;
preferably, the average diameter of the microsphere body is 200 nm, 250 nm, 300 nm, 350 nm, 400 nm, 450 nm, 500 nm, 550 nm, 600 nm, 650 nm, 700 nm, 750 nm, 800 nm, 850 nm, 900 nm, 950 nm, or 1000 nm, with a deviation of ±20%, more preferably ±10%.

8. The DNA microsphere according to any one of claims 1-7, wherein the polymer is one or more of polyolefin, PEG, polyacrylic acid, polyamide, or polyester.

9. The DNA microsphere according to any one of claims 1-8, wherein the average molecular weight (MW) of the polymer is 1000-5000000; more preferably, the MW is 1000-100000; further preferably, the MW is 2000-50000; still further preferably, the MW is 3000-10000.

10. The DNA microsphere according to any one of claims 1-9, wherein the DNA is obtained by amplification using a DNA primer sequence, preferably, the length of the DNA primer sequence is 6-30 bp, preferably 9-21 bp; further preferably, the amplification is performed following the binding of the DNA primer sequence to the polymer via the functional group prior to the amplification, further, the DNA microsphere is obtained by connecting the polymer bound to the DNA primer sequence via the functional group after the amplification to the surface of the microsphere body.

11. The DNA microsphere according to claim 10, wherein the DNA primer sequence is modified with the functional group; further, the functional group is an amino group, and the DNA primer sequence contains an amino group at the 5' end; preferably, the amino group is contained at C2-C20 position of the 5' end; more preferably, the amino group is contained at the C5-C10 position of the 5' end; most preferably, the amino group is contained at the C6 position of the 5' end.

12. A preparation method for the DNA microsphere according to any one of claims 1-11, comprising the following steps: (1) chemically modifying the microsphere body with an aminated silane coupling agent to introduce an amino group onto the outer surface of the microsphere body, thereby forming an amino-modified microsphere A; (2) modifying a DNA primer sequence to obtain a modified DNA primer sequence; (3) binding the modified DNA primer sequence to a polymer, followed by amplification to obtain a polymer-modified DNA; (4) reacting the polymer-modified DNA with the amino-modified microsphere A to obtain the DNA microsphere.

13. The preparation method for the DNA microsphere according to claim 13, wherein in step (2), the DNA primer sequence is modified with the amino group at the 5' end; preferably, the DNA primer sequence is modified with the amino group at the C2-C20 position of the 5' end; more preferably, the DNA primer sequence is modified with the amino group at the C5-C10 position of the 5' end; most preferably, the DNA primer sequence is modified with the amino group at the C6 position of the 5' end.

14. The preparation method for the DNA microsphere according to claim 12 or 13, wherein the length of the DNA primer sequence is 6-30 bp; preferably 9-21 bp, further, the DNA primer sequence is GGCGTAGAGGAT.

15. The preparation method for the DNA microsphere according to any one of claims 12-14, wherein the polymer is a polyolefin polymer; preferably, the polyolefin polymer is selected from one or more of substituted or unsubstituted polypropylene, polyacrylic acid, polyacrylate salt, polyacrylate ester, or polyacrylamide.

16. The preparation method for the DNA microsphere according to any one of claims 12-15, wherein in step (4), before reacting the polymer-modified DNA with the microsphere A, the system containing the microsphere A is subjected to ultrasonic concussion.

17. The preparation method for the DNA microsphere according to claim 16, wherein the duration of the ultrasonic concussion is 1-30 min, preferably 5-20 min; more preferably 5-10 min.

18. Use of the DNA microsphere according to any one of claims 1-11, wherein the DNA microsphere is used as a DNA template.

19. Use of the DNA microsphere according to any one of claims 1-11 as a DNA template in RNA transcription or protein synthesis.

20. The use according to claim 20, wherein the RNA transcription or protein synthesis is in vitro RNA or in vitroprotein synthesis.

21. A cell-free *in* vitro synthesis system, comprising: (1) a cell extract; (2) the DNA microsphere according to any one of claims 1-11.

22. A method for cell-free in *vitro* synthesis, comprising utilizing the system according to claim 21 for preparation.
